# EUROPEAN PATENT SPECIFICATION

(11) **EP 2 576 601 B1**
(45) Date of publication and mention of the grant of the patent: **23.11.2016**
(21) Application number: 11727557.8
(22) Date of filing: 03.06.2011
(51) Int. Cl.: C07K 14/245, C12N 9/14, C12P 13/10, C12P 13/14

(54) **A METHOD FOR PRODUCING AN L-AMINO ACID USING A BACTERIUM OF THE ENTEROBACTERIACEÆ FAMILY, HAVING ATTENUATED EXPRESSION OF GENES ENCODING A LYSINE/ARGININE/ORNITHINE TRANSPORTER**
VERFAHREN ZUR HERSTELLUNG EINER L-AMINOSÄURE UNTER VERWENDUNG EINES BAKTERIUMS DER FAMILIE ENTEROBACTERIACEAE MIT ABGESCHWÄCHTER EXPRESSION VON FÜR EINEN PLYSIN/ARGININ/ORNITHINE-TRANSPORTER CODIERENDEN GENEN
PROCÉDÉ DE PRODUCTION D'UN ACIDE L AMINÉ À L'AIDE D'UNE BACTÉRIE DE LA FAMILLE DES ENTÉROBACTÉRIACÉS, AFFICHANT UNE EXPRESSION ATTÉNUÉE DE GÈNES CODANT POUR UN TRANSPORTEUR DE LYSINE/ARGININE/ORNITHINE

(30) Priority: 03.06.2010 RU 2010122646
(43) Date of publication of application: 10.04.2013
(73) Proprietor: Ajinomoto Co., Inc., Tokyo 104-8315 (JP)
(72) Inventor: ROSTOVA, Yulia Georgievna, Moscow 129642 (RU); VOROSHILOVA, Elvira Borisovna, Moscow 117648 (RU); GUSYATINER, Mikhail Markovich, Moscow 117648 (RU)
(74) Representative: Strehl Schübel-Hopf & Partner
(86) International application number: PCT/JP2011/063304
(87) International publication number: WO 2011/152568

(56) References cited:
- EP-A1- 2 196 535
- US-A1- 2010 143 983
- XIE ET AL: "Transcriptome of Escherichia coli K1 bound to human brain microvascular endothelial cells", BIOCHEMICAL AND BIOPHYSICAL RESEARCH COMMUNICATIONS, ACADEMIC PRESS INC. ORLANDO, FL, US, vol. 365, no. 1, 5 November 2007 (2007-11-05), pages 201-206, XP022349653, ISSN: 0006-291X, DOI: 10.1016/J.BBRC.2007.10.174
- WISSENBACH U ET AL: "A third periplasmic transport system for L-arginine in Escherichia coli: Molecular characterization of the artPIQMJ genes, arginine binding and transport", MOLECULAR MICROBIOLOGY, WILEY-BLACKWELL PUBLISHING LTD, GB, vol. 17, no. 4, 1 January 1995 (1995-01-01), pages 675-686, XP002572975, ISSN: 0950-382X, DOI: 10.1111/J.1365-2958.1995.MMI_17040675.X
- M. Caldara ET AL: "The arginine regulon of Escherichia coli: whole-system transcriptome analysis discovers new genes and provides an integrated view of arginine regulation", Microbiology, vol. 152, no. 11, 1 November 2006 (2006-11-01), pages 3343-3354, XP055180916, ISSN: 1350-0872, DOI: 10.1099/mic.0.29088-0

## Description

### Technical Field

The present invention relates to the microbiological industry, and specifically to a method for producing an L-amino acid using a bacterium of the *Enterobacteriaceae* family in which expression of one or more genes encoding a lysine/arginine/ornithine transporter are attenuated.

### Background Art

Conventionally, L-amino acids are industrially produced by fermentation methods utilizing strains of microorganisms obtained from natural sources, or mutants thereof. Typically, the microorganisms are modified to enhance production yields of L-amino acids.

Many techniques to enhance L-amino acid production yields have been reported, including by transforming of microorganisms with recombinant DNA (see, for example, US patent No. 4,278,765). Other techniques for enhancing production yields include increasing the activities of enzymes involved in amino acid biosynthesis and/or desensitizing the target enzymes of the feedback inhibition caused by the resulting L-amino acid (see, for example, WO 95/16042 or US patent Nos. 4,346,170; 5,661,012 and 6,040,160).

Another way to enhance L-amino acid production yields is to attenuate expression of a gene or several genes involved in the degradation of the target L-amino acid, genes diverting the precursors of the target L-amino acid from the L-amino acid biosynthetic pathway, genes involved in the redistribution of carbon, nitrogen, and phosphate fluxes, and genes coding for toxins etc.

The *hisJ* and *argT* genes of *Salmonella typhimurium* encode two periplasmic binding proteins, J and LAO, which are involved in histidine and arginine transport, respectively, and which interact with a common membrane-bound component, the P protein. The complete nucleotide sequences of these two genes have been determined. The two genes show extensive homology (70%) and presumably arose by tandem duplication of a single ancestral gene. The two encoded proteins now perform distinct functions but still retain sufficient homology to permit interaction with the same site on the membrane-bound P protein (Higgins C.F., Ames G.F., Proc Natl Acad Sci U S A.;78(10):6038-42(1981)).

The superfamily of traffic ATPases (ABC transporters) includes bacterial periplasmic transport systems (permeases) and various eukaryotic transporters. The histidine permease *of S. typhimurium* and *Escherichia coli* is composed of a soluble receptor including a membrane-bound complex containing four subunits, the substrate-binding protein (HisJ), and is energized by ATP. It was shown that histidine transport depends entirely on both ATP and the ligand HisJ, and is affected by pH, temperature, and salt concentration. Transport is irreversible and accumulation reaches a plateau at which point transport ceases. The permease is inhibited by ADP and by high concentrations of internal histidine. The inhibition by histidine likely indicates that the membrane-bound complex HisQ/M/P includes a substrate-binding site. The reconstituted permease activity corresponds to an about 40-70% turnover rate relative to the in vivo rate of transport (Liu C.E., Ames G.F., J Biol Chem.; 272(2):859-66(1997)).

The membrane-bound complex of the *Salmonella typhimurium* periplasmic histidine permease is composed of two integral membrane proteins, HisQ and HisM, and two copies of an ATP-binding subunit, HisP. The complex hydrolyzes ATP upon induction of the activity by the soluble receptor and the periplasmic histidine-binding protein, HisJ. It was shown that the nucleotide-binding component can be stripped off of the integral membrane components, HisQ and HisM. The complex can be reconstituted by using the HisP-depleted membranes containing HisQ and HisM and pure soluble HisP. HisP was shown to have high affinity for the HisP-depleted complex, HisQM, and two HisP molecules are recruited independently of each other for each HisQM unit. The in vitro reassembled complex has entirely normal properties, and responds to the HisJ and ATPase inhibitors with the same characteristics as the original complex and in contrast to those of soluble HisP. These results show that HisP is absolutely required for ATP hydrolysis, HisQM cannot hydrolyze ATP, HisP depends on HisQM to relay the inducing signal from the soluble receptor HisJ, and HisQM regulates the ATPase activity of HisP. It was also shown that HisP changes conformation upon exposure to phospholipids (Liu P.Q., Ames G.F., Proc Natl Acad Sci U S A.; 95(7):3495-500(1998)).

The ArgT protein *of E. coli* can be a cytoplasmic precursor protein of 28 kDa and a mature periplasmic protein of 25.8 kDa. To elucidate the involvement of proteolysis in the regulation of stationary-phase adaptation, the clpA, clpX, and clpP protease mutants of *Escherichia coli* were subjected to proteome analysis during growth and carbon starvation. The periplasmic lysine-arginine-ornithine binding protein ArgT in the clpA, clpX, or clpP mutant did not display induction typical of late-stationary-phase wild-type cells (Weichart D. et al., Journal of Bacteriology, Vol. 185, No. 1, p. 115-125(2003)).

But currently, there have been no reports of attenuating expression of genes encoding a lysine/arginine/ornithine transporter for the purpose of producing L-amino acids. M. Caldara et al.: "The arginine regulon of Escherichia coli; whole-system transcriptome analysis discovers new genes and provides an integrated view of arginine regulation" Microbiology, vol. 152, no. 11, 1 November 2006, pages 3343-3354 discloses that the expression of the hisJQMP operon is repressed by arginine.

### Disclosure of the Invention

Aspects of the present invention include enhancing the productivity of L-amino acid-producing strains and providing a method for producing an L-amino acid using these strains.

It has been found that attenuating expression of the arg*T-hisJQMP* cluster can enhance production of amino acids, for example, diaminomonocarboxylic acids, such as L-lysine, L-arginine, ornithine, and citrulline.

The present invention provides the following.
[1] A method for producing an L-amino acid comprising:
   - cultivating an L-amino acid-producing bacterium of the *Enterobacteriaceae* family in a medium, and
   - collecting said L-amino acid from the medium,
      wherein said bacterium has been modified to attenuate expression of one or more genes encoding a lysine/arginine/ornithine transporter, said one or more genes encoding the lysine/arginine/ornithine transporter being one or more genes of *argT-hisJQMP* cluster, and
      wherein said bacterium is able to produce and cause accumulation of the L-amino acid in a culture medium in an amount larger than a wild-type or parental strain.
[2] The method according to [1]above, wherein said one or more genes of the *argT*-*hisJQMP* cluster is/are inactivated.
[3] The method according to [1] or [2] above, wherein said bacterium belongs to genus *Escherichia.*
[4] The method according to [3] above, wherein said bacterium is *Escherichia coli.*
[5] The method according to [1] or [2] above, wherein said bacterium belongs to genus *Pantoea.*
[6] The method according to any of [1] to [5] above, wherein said L-amino acid is a diaminomonocarboxylic acid.
[7] The method according to [6] above, wherein said diaminomonocarboxylic acid is selected from the group consisting of L-lysine, L-arginine, L-ornithine, and L-citrulline.

### Description of the Embodiments

The present invention is described in detail below.

### 1. Bacterium

The bacterium in accordance with the presently disclosed subject matter is a bacterium of the *Enterobacteriaceae* family which is able to produce an L-amino acid such as a diaminomonocarboxylic acid, wherein the baccterium has been modified to attenuate expression of one or more genes encoding the lysine/arginine/ornithine transporter.

The term "bacterium producing an L-amino acid" means a bacterium which is able to produce and cause accumulation of an L-amino acid such as diaminomonocarboxylic acid in a culture medium in an amount larger than a wild-type or parental strain *of E. coli,* such as *E. coli* K-12.

The term "diaminomonocarboxylic acid" can include L-lysine, L-arginine, ornithine, and citrulline. These amino acids have a positive charge due to the presence of an amino group.

The *Enterobacteriaceae* family includes bacteria belonging to the genera *Escherichia, Enterobacter, Erwinia, Klebsiella, Pantoea, Photorhabdus, Providencia, Salmonella, Serratia, Shigella, Morganella, Yersinia,* etc.. Specifically, those classified into the *Enterobacteriaceae* according to the taxonomy used by the NCBI (National Center for Biotechnology Information) database (http://www.ncbi.nlm.nih.gov/Taxonomy/Browser/wwwtax.cgi?id=543) can be used. A bacterium belonging to the genus *Escherichia* or *Pantoea* is preferred.

The phrase "a bacterium belonging to the genus *Escherichia"* can mean that the bacterium is classified into the genus *Escherichia* according to the classification known to a person skilled in the art of microbiology. Examples of a bacterium belonging to the genus *Escherichia* include *Escherichia coli* (*E. coli*).

The bacterium belonging to the genus *Escherichia* is not particularly limited; however, e.g., bacteria described by Neidhardt, F.C. et al. (Escherichia coli and Salmonella typhimurium, American Society for Microbiology, Washington D.C., 1208, Table 1) can be used.

The phrase "a bacterium belonging to the genus *Pantoea"* can mean that the bacterium is classified into the genus *Pantoea* according to the classification known to a person skilled in the art of microbiology. Some species of *Enterobacter agglomerans* have been recently re-classified into *Pantoea agglomerans, Pantoea ,ananatis, or Pantoea stewartii,,* based on the nucleotide sequence analysis of 16S rRNA, etc. (Int. J. Syst. Bacteriol., 43, 162-173 (1993)).

The phrase "expression of one or more genes encoding a lysine/arginine/ornithine transporter is attenuated" means that the bacterium has been modified in such a way that the modified bacterium contains reduced amounts of the lysine/arginine/ornithine transporter as compared with an unmodified bacterium, or the modified bacterium is unable to synthesize the lysine/arginine/ornithine transporter.

The phrase "inactivation of a gene" can mean that the modified gene encode completely inactive protein. It is also possible that the modified DNA region is unable to naturally express the gene due to the deletion of the gene, the shifting of the reading frame of the gene, the introduction of missense/nonsense mutation(s), or the modification of an adjacent region of the gene, including sequences controlling gene expression, such as promoter(s), enhancer(s), attenuator(s), ribosome-binding site(s), etc..

The presence or absence of a target gene on the chromosome of a bacterium can be detected by well-known methods, including *PCR and Southern blotting.*

In addition, the level of expression of the gene can be estimated by measuring the amount of mRNA transcribed from the gene using various well-known methods, including Northern blotting and quantivative RT-PCR. The amount or molecular weight of the protein encoded by the target gene can be measured by well-known methods, including SDS-PAGE followed by immunoblotting blotting analysis) .

Examles of one or more genes encoding the lysine/arginine/ornithine transporter include genes constituting the *argT-hisJQMP* cluster.

The *argT* gene (synonyms - *ECK2304, b2310*) encodes ArgT, which is a subunit of the lysine/arginine/ornithine ABC transporter (synonym - B2310). The *argT* gene of *E. coli* (nucleotides complementary to nucleotides 2,425,031 to 2,425,813 in the GenBank accession number NC_000913.2; gi:16130244) is located between the gene *hisJ* and the gene *ubiX*, both oriented in the same directions as *argT,* on the chromosome *of E. coli* strain K-12. The nucleotide sequence of the *argT* gene and the amino acid sequence of ArgT encoded by the *argT* gene are shown in SEQ ID NO: 1 and SEQ ID NO: 2, respectively.

The *hisJ* gene (synonyms *- ECK2303, b2309)* encodes HisJ, which is a subunit of the histidine ABC transporter (synonym - B2309). The *hisJ* gene of *E. coli* (nucleotides complementary to nucleotides 2,424,028 to 2,424,810 in the GenBank accession number NC_000913.2; gi: 16130244) is located between the gene *argT* and the gene *hisQ,* both oriented in the same directions as *hisJ,* on the chromosome *of E. coli* strain K-12. The nucleotide sequence of the *hisJ* gene and the amino acid sequence of HisJ encoded by the *hisJ* gene are shown in SEQ ID NO: 3 and SEQ ID NO: 4, respectively.

Attenuation of expression of the *hisJ* gene is not essential for the presetn invetnion, but expression the *hisJ* gene can be attenuated.

The *hisQ* gene (synonyms - *ECK2302*, *b2308)* encodes HisQ, which is a subunit of the lysine/arginine/ornithine ABC transporter (synonym - B2308). The *hisQ* gene *of E. coli* (nucleotides complementary to nucleotides 2,423,252 to 2,423,938 in the GenBank accession number NC_000913.2; gi: 16130243) is located between the gene *hisJ* and the gene *hisM,* both oriented in the same directions as *hisQ,* on the chromosome of *E*. *coli* strain K-12. The nucleotide sequence of the *hisQ* gene and the amino acid sequence of HisQ encoded by the *hisQ* gene are shown in SEQ ID NO: 5 and SEQ ID NO: 6, respectively.

The *hisM* gene (synonyms - *ECK2301, b2307*) encodes HisM, which is a subunit of the lysine/arginine/ornithine ABC transporter (synonym - B2307). The *hisM* gene *of E. coli* (nucleotides complementary to nucleotides 2,422,539 to 2,423,255 in the GenBank accession number NC_000913.2; gi: 16130242) is located between the gene *hisQ* and the gene *hisP,* both oriented in the same directions as *hisM,* on the chromosome of *E*. *coli* strain K-12. The nucleotide sequence of the *hisM* gene and the amino acid sequence of HisM encoded by the *hisM* gene are shown in SEQ ID NO: 7 and SEQ ID NO: 8, respectively.

The *hisP* gene (synonyms - *ECK2300, b2306*) encodes HisP, which is a subunit of the lysine/arginine/ornithine ABC transporter (synonym - B2306). The *hisP* gene *of E. coli* (nucleotides complemented to nucleotides 2,421,758 to 2,422,531 in the GenBank accession number NC_000913.2; gi: 16130241) is located between the gene *hisM* and the ORF *yfcI,* both oriented in the same directions as *hisP,* on the chromosome *of E. coli* strain K-12. The nucleotide sequence of the *hisP* gene and the amino acid sequence of HisP encoded by the *hisP* gene are shown in SEQ ID NO: 9 and SEQ ID NO: 10, respectively.

Since there may be some differences in DNA sequences between the genera or strains of the *Enterobacteriaceae* family, the *argT-hisJQMP* cluster is not limited to the genes shown in SEQ ID NO: 1, SEQ ID NO: 3, SEQ ID NO: 5, SEQ ID NO: 7 and SEQ ID NO: 9, but may include genes homologous to SEQ ID NO: 1, SEQ ID NO: 3, SEQ ID NO: 5, SEQ ID NO: 7 and SEQ ID NO: 9 which encode variant proteins of the ArgT, HisJ, HisQ, HisM and HisP. The phrase "variant protein" can mean a protein which has changes in the sequence, whether they are deletions, insertions, additions, or substitutions of amino acids, but the function of the protein is maintained. The number of changes in the variant protein depends on the position in the three dimensional structure of the protein or the type of amino acid residues. It may be 1 to 30, or in another example 1 to 15, and or in another example 1 to 5 in SEQ ID NO: 2, SEQ ID NO: 4, SEQ ID NO: 6, SEQ ID NO: 8 and SEQ ID NO: 10. These changes can occur in regions of the protein which are not critical for the function of the protein. This is because some amino acids have high homology to one another so the three dimensional structure or activity is not affected by such a change. Therefore, the protein variant encoded by the gene of *argT-hisJQMP* cluster may be one which has a homology of not less than 80%, or in another example not less than 90%, not less than 95%, in another example not less than 98%, and in another example not less than 99%, with respect to the entire amino acid sequence shown in SEQ ID NO: 2, SEQ ID NO: 4, SEQ ID NO: 6, SEQ ID NO: 8 or SEQ ID NO: 10, as long as the functioning of the protein is maintained. In this specification, the term "homology" can also refer to "identity".

Homology between two amino acid sequences can be determined using well-known methods, for example, the computer program BLAST 2.0, which calculates three parameters: score, identity and similarity.

Moreover, the gene of the *argT-hisJQMP* cluster may be a variant which hybridizes under stringent conditions with the nucleotide sequence shown in SEQ ID NO: 1, SEQ ID NO: 3, SEQ ID NO: 5, SEQ ID NO: 7 or SEQ ID NO: 9, or a probe which can be prepared from the nucleotide sequence, under stringent conditions, provided that it encodes a functional protein. "Stringent conditions" include those under which a specific hybrid, for example, a hybrid having homology of not less than 60%, or in another example not less than 70%, or in another example not less than 80%, or in another example not less than 90%, or in another example not less than 95%, in another example not less than 98%, or in another example not less than 99%is formed and a non-specific hybrid, for example, a hybrid having homology lower than the above, is not formed. For example, stringent conditions are exemplified by washing one time or more, or in another example two or three times at a salt concentration of 1 X SSC, 0.1% SDS, preferably 0.1 X SSC, 0.1% SDS at 60°C. Duration of washing depends on the type of membrane used for blotting and, as a rule, should be what is recommended by the manufacturer. For example, the recommended duration of washing for the Hybond™ N+ nylon membrane (Amersham) under stringent conditions is 15 minutes. Washing may be performed 2 to 3 times. The length of the probe may be suitably selected depending on the hybridization conditions, and is usually 100 bp to 1 kbp.

Expression of the gene of the *argT-hisJQMP* cluster can be attenuated by introducing mutations into the genes so that the intracellular activity of the protein encoded by the gene is decreased as compared with an unmodified strain. Such a mutation on the gene can be a replacement of one base or more to cause amino acid substitution in the protein encoded by the gene (missense mutation), introduction of a stop codon (nonsense mutation), deletion of one or two bases to cause a frame shift, insertion of a drug-resistance gene, or deletion of a part of the gene or the entire gene (Qiu, Z. and Goodman, M.F., J. Biol. Chem., 272, 8611-8617 (1997); Kwon, D. H. et al, J. Antimicrob. Chemother., 46, 793-796 (2000)). Expression of the gene of the *argT-hisJQMP* cluster can also be attenuated by modifying an expression regulating sequence such as the promoter, the Shine-Dalgarno (SD) sequence, etc. of each gene (WO95/34672, Carrier, T.A. and Keasling, J.D., Biotechnol Prog 15, 58-64 (1999)).

For example, the following methods may be employed to introduce a mutation by gene recombination. A mutant gene encoding a mutant protein with decreased activity can be prepared, and a bacterium to be modified can be transformed with a DNA fragment containing the mutant gene. Then, the native gene on the chromosome is replaced with the mutant gene by homologous recombination, and the resulting strain can be selected. Such gene replacement by homologous recombination can be conducted by employing a linear DNA, which is known as "Red-driven integration" (Datsenko, K.A. and Wanner, B.L., Proc. Natl. Acad. Sci. USA, 97, 12, p 6640-6645 (2000), WO2005/010175), or by methods employing a plasmid containing a temperature-sensitive replication control region (Proc. Natl. Acad. Sci., USA, 97, 12, p 6640-6645 (2000), U.S. Patent Nos. 6,303,383 and 5,616,480). Furthermore, the introduction of a site-specific mutation by gene replacement using homologous recombination as set forth above can also be performed by using a plasmid which is unable to replicate in the host.

Expression of the target gene can also be attenuated by insertion of a transposon or an IS factor into the coding region of the gene (U.S. Patent No. 5,175,107), or by conventional methods, such as mutagenesis treatment using UV irradiation or nitrosoguanidine (N-methyl-N'-nitro-N-nitrosoguanidine) treatment.

Inactivation of the target gene can also be performed by conventional methods, such as a mutagenesis treatment using UV irradiation or nitrosoguanidine (N-methyl-N'-nitro-N-nitrosoguanidine) treatment, site-directed mutagenesis, gene disruption using homologous recombination, or/and insertion-deletion mutagenesis (Yu, D. et al., Proc. Natl. Acad. Sci. USA, 2000, 97:12: 5978-83 and Datsenko, K.A. and Wanner, B.L., Proc. Natl. Acad. Sci. USA, 2000, 97:12: 6640-45) also called "Red-driven integration".

Methods for preparation of plasmid DNA, digestion and ligation of DNA, transformation, and selection of an oligonucleotide as a primer may be ordinary methods well-known to one skilled in the art. These methods are described, for instance, in Sambrook, J., Fritsch, E.F., and Maniatis, T., "Molecular Cloning: A Laboratory Manual, Second Edition", Cold Spring Harbor Laboratory Press (1989).

### L-amino acid-producing bacteria

The bacteria in which expression is attenuated of one or more genes encoding the lysine/arginine/omithine transporter can be bacteria which are able to produce L-amino acids such as diaminomonocarboxylic acid may be used in the method in accordance with the presently disclosed subject matter.

The bacterium can be obtained by attenuating expression of the gene/-s encoding the lysine/arginine/ornithine transporter in a bacterium which inherently has the ability to produce L-amino acids such as diaminomonocarboxylic acid. Alternatively, the bacterium can be obtained by imparting the ability to produce L-amino acids such as diaminomonocarboxylic acid to a bacterium in which expression of the gene/-s encoding the lysine/arginine/ornithine transporter has already been attenuated .

### L-lysine-producing bacteria

Examples of L-lysine-producing bacteria or parent strains belonging to the genus *Escherichia* include mutants having resistance to an L-lysine analogue. The L-lysine analogue inhibits growth of bacteria belonging to the genus *Escherichia,* but this inhibition is fully or partially desensitized when L-lysine is present in the medium. Examples of the L-lysine analogue include oxalysine, lysine hydroxamate, S-(2-aminoethyl)-L-cysteine (AEC), γ-methyllysine, α-chlorocaprolactam, and so forth. Mutants having resistance to these lysine analogues can be obtained by subjecting bacteria belonging to the genus *Escherichia* to a conventional artificial mutagenesis treatment. Specific examples of bacterial strains useful for producing L-lysine include *Escherichia coli* AJ11442 (FERM BP-1543, NRRL B-12185; see U.S. Patent No. 4,346,170) and *Escherichia coli* VL611. In these microorganisms, feedback inhibition of aspartokinase by L-lysine is desensitized.

The strain WC196 may be used as an L-lysine producing bacterium of *Escherichia coli.* This bacterial strain was bred from the W3110 strain, which was derived from *Escherichia coli* K-12, by replacing the wild type *lysC* gene on the chromosome of the W3110 strain with a mutant *lysC* gene encoding a mutant aspartokinase III desensitized to feedback inhibition by L-lysine in which threonine at position 352 had been replaced with isoleucine, and conferring AEC resistance to the resulting strain (U.S. patent No. 5,661,012). The resulting strain was designated *Escherichia coli* AJ13069 and was deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6, 1994 and received an accession number of FERM P-14690. Then, it was converted to an international deposit under the provisions of the Budapest Treaty on September 29, 1995, and received an accession number of FERM BP-5252 (U.S. Patent No. 5,827,698).

Examples of parent strains which can be used to derive bacteria which are able to produce L-lysine also include strains in which expression of one or more genes encoding an L-lysine biosynthetic enzyme are enhanced. Examples of such genes include genes encoding dihydrodipicolinate synthase (*dapA*), aspartokmase (*lysC*), dihydrodipicolinate reductase (*dapB*), diaminopimelate decarboxylase (*lysA*), diaminopimelate dehydrogenase *(ddh)* (U.S. Patent No. 6,040,160), phosphoenolpyrvate carboxylase (*ppc*)*,* aspartate semialdehyde dehydrogenease (*asd*), and aspartase (*aspA*) (EP 1253195 A). In addition, the parent strains may have increased expression of the gene involved in energy efficiency (*cyo*) (EP 1170376 A), the gene encoding nicotinamide nucleotide transhydrogenase (*pntAB*) (U.S. Patent No. 5,830,716), the *ybjE* gene (WO2005/073390), or combinations thereof.

Examples of parent strains which can be used to derive bacteria that are able to produce L-lysine also include strains having decreased or no activity of an enzyme that catalyzes a reaction for generating a compound other than L-lysine by branching off from the biosynthetic pathway of L-lysine. Examples of the enzymes that catalyze a reaction for generating a compound other than L-lysine by branching off from the biosynthetic pathway of L-lysine include homoserine dehydrogenase, lysine decarboxylase (U.S. Patent No. 5,827,698), and the malic enzyme (WO2005/010175).

### L-arginine-producing bacteria

Examples of parent strains which can be used to derive bacteria that are able to produce L-arginine include strains belonging to the genus *Escherichia,* such as *E. coli* strain 237 (VKPM B-7925) (U.S. Patent Application 2002/058315 A1) and its derivative strains harboring mutant N-acetylglutamate synthase (Russian Patent Application No. 2001112869), *E. coli* strain 382 (VKPM B-7926) (EP1170358A1), and an arginine-producing strain into which the *argA* gene encoding N-acetylglutamate synthetase is introduced therein (EP1170361A1).

Examples of parent strains which can be used to derive bacteria that can produce L-arginine also include strains in which expression of one or more genes encoding an L-arginine biosynthetic enzyme are enhanced. Examples of such genes include genes encoding N-acetylglutamyl phosphate reductase (*argC*), ornithine acetyl transferase (*argJ*)*,* N-acetylglutamate kinase (*argB*), acetylornithine transaminase (*argD*), ornithine carbamoyl transferase (*argF*), argininosuccinic acid synthetase (*argG*), argininosuccinic acid lyase (*argH*)*,* and carbamoyl phosphate synthetase (*carAB*).

### L-citrulline producing bacteria

Examples of L-citrulline-producing bacteria or parent strains which can be used to derive bacteria that are able to produce citrulline include strains belonging to the genus *Escherichia,* such as *E.coli* mutant *N*-Acetylglutamate Synthase strains 237/pMADS11, 237/pMADS12 and 237/pMADS13 (RU2215783, EP1170361B1, US6790647B2).

Also, bacteria which produce citrulline can be easily obtained from any bacterium which is able to produce arginine, for example *E. coli* stain 382 (VKPM B-7926), by inactivation of argininosuccinate synthase encoded by the *argG* gene.

The phrase "inactivation of argininosuccinate synthase" means that the bacterium has been modified in such a way that the modified bacterium contains an inactive argininosuccinate synthase, or it can also mean that the bacterium is unable to synthesize the argininosuccinate synthase. Inactivation of argininosuccinate synthase can be performed by inactivation of the *argG* gene.

The phrase "inactivation of the *argG* gene" means that the modified gene encodes a completely non-functional protein. It is also possible that the modified DNA region is unable to naturally express the gene due to a deletion of a part of the gene or the whole gene, the shifting of the reading frame of the gene, the introduction of missense/nonsense mutation(s), or the modification of an adjacent region of the gene, including sequences controlling gene expression, such as a promoter, enhancer, attenuator, ribosome-binding site, etc..

The presence or absence of the *argG* gene on the chromosome of a bacterium can be detected by well-known methods, including PCR, Southern blotting.

In addition, the level of gene expression can be estimated by measuring the amount of mRNA transcribed from the gene using various well-known methods, including Northern blotting and quantitative RT-PCR. The amount of the protein encoded by the *argG* gene can be measured by well-known methods, includi SDS-PAGE followed by an iinmunoblotting assay (Western blotting analysis).

Expression of the *argG* gene can be attenuated by introducing a mutation into the gene on the chromosome so that the intracellular activity of the protein encoded by the gene is decreased as compared with an unmodified strain. Mutations which result in attenuation of expression of the gene include the replacement of one base or more to cause an amino acid substitution in the protein encoded by the gene (missense mutation), introduction of a stop codon (nonsense mutation), deletion of one or two bases to cause a frame shift, insertion of a drug-resistance gene, or deletion of a part of the gene or the entire gene (Qiu, Z. and Goodman, M.F., J. Biol. Chem., 272, 8611-8617 (1997); Kwon, D. H. et al, J. Antimicrob. Chemother., 46, 793-796 (2000)). Expression of the *argG* gene can also be attenuated by modifying an expression regulating sequence such as the promoter, the Shine-Dalgarno (SD) sequence, etc. (WO95/34672, Carrier, T.A. and Keasling, J.D., Biotechnol Prog 15, 58-64 (1999)).

For example, the following methods may be employed to introduce a mutation by gene recombination. A mutant gene encoding a mutant protein with decreased activity is prepared, and the bacterium is transformed with a DNA fragment containing the mutant gene. Then, the native gene on the chromosome is replaced with the mutant gene by homologous recombination, and the resulting strain is selected. Gene replacement or disruption using homologous recombination can be conducted by employing a linear DNA, which is known as "Red-driven integration" (Datsenko, K.A. and Wanner, B.L., Proc. Natl. Acad. Sci. USA, 97, 12, p 6640-6645 (2000)), or by employing a plasmid containing a temperature-sensitive replication origin (U.S. Patent 6,303,383 or JP 05-007491A). Furthermore, site-specific mutation by gene substitution can also be incorporated using homologous recombination such as set forth above using a plasmid that is unable to replicate in the host.

Expression of the gene can also be attenuated by inserting a transposon or an IS factor into the coding region of the gene (U.S. Patent No. 5,175,107), or by conventional methods, such as by mutagenesis with UV irradiation or nitrosoguanidine (N-methyl-N'-nitro-N-nitrosoguanidine).

### L-ornithine producing bacteria

Bacteria which are able to produce ornithine can be easily obtained from any arginine-producing bacteria, for example *E*. *coli* stain 382 (VKPM B-7926), by inactivation of ornithine carbamoyltransferase encoded by both the *argF* and *argI* genes. Inactivation of ornithine carbamoyltransferase can be performed in the same way as described above.

### 2. Method of the present invention

Exemplary method in accordance with the presently disclosed subject matter includes production of an L-amino acid by cultivating a bacterium in a culture medium to produce and excrete the L-amino acid into the medium, and collecting the L-amino acid from the medium.

The cultivation, collection, and purification of an L-amino acid from the medium may be performed in a manner similar to conventional fermentation methods wherein an amino acid is produced using a bacterium.

The medium used for culture may be either a synthetic or natural medium, so long as the medium includes a carbon source and a nitrogen source and minerals and, if necessary, appropriate amounts of nutrients which the chosen bacterium requires for growth. The carbon source may include various carbohydrates such as glucose and sucrose, and various organic acids. Depending on the mode of assimilation of the chosen microorganism, alcohol, including ethanol and glycerol, may be used. As the nitrogen source, various ammonium salts such as ammonia and ammonium sulfate, other nitrogen compounds such as amines, a natural nitrogen source such as peptone, soybean-hydrolysate, and digested fermentative microorganism can be used. As minerals, potassium monophosphate, magnesium sulfate, sodium chloride, ferrous sulfate. manganese sulfate, and calcium chloride can be used. As vitamins, thiamine, and yeast extract can be used.

The cultivation can be performed under aerobic conditions, such as by shaking, and/or by stirring with aeration, at a temperature of 20 to 40 °C, or in another example 30 to 38 °C. The pH of the culture is usually between 5 and 9, or in another example between 6.5 and 7.2. The pH of the culture can be adjusted with ammonia, calcium carbonate, various acids, various bases, and buffers. Usually, a 1 to 5-day cultivation leads to accumulation of the target L-amino acid in the liquid medium.

After cultivation, solids such as cells can be removed from the liquid medium by centrifugation or membrane filtration, and then the L-amino acid can be collected and purified by ion-exchange, concentration, and/or crystallization methods.

### Examples

The present invention will be more concretely explained below with reference to the following Examples.

### Example 1. Construction of a strain with an inactivated argT-hisJOMP cluster.

### 1. Deletion of the argT-hisJQMP cluster.

A strain in which the *argT-hisJQMP* cluster is deleted was constructed by the method initially developed by Datsenko, K.A. and Wanner, B.L. (Proc. Natl. Acad. Sci. USA, 2000, 97(12), 6640-6645) called "Red-driven integration". The DNA fragment containing the Cm^{R} marker encoded by the *cat* gene was obtained by PCR, using primers P1 (SEQ ID NO: 11) and P2 (SEQ ID NO: 12), and plasmid pMW118-attL-Cm-attR as the template (WO 05/010175). Primer P1 contains both a region complementary to the region located at the 5' end of the *argT* gene and a region complementary to the attR region. Primer P2 contains both a region complementary to the region located at the 3' end of the *hisP* gene and a region complementary to the attL region. The conditions for PCR were as follows: denaturation step for 3 min at 95 °C; profile for two first cycles: 1 min at 95 °C, 30 sec at 50 °C, 40 sec at 72 °C; profile for the last 25 cycles: 30 sec at 95 °C, 30 sec at 54 °C, 40 sec at 72 °C; final step: 5 min at 72 °C.

A 1.6 kbp PCR product was obtained and purified in agarose gel and was used for electroporation of the *E. coli* strain MG1655 (ATCC 700926), which contains the plasmid pKD46. The plasmid pKD46 (Datsenko, K.A. and Wanner, B.L., Proc. Natl. Acad. Sci. USA, 2000, 97:12:6640-45) contains a temperature-sensitive replication origin, and includes a 2,154 nucleotide DNA fragment of phage λ (nucleotide positions 31088 to 33241, GenBank accession no. J02459), as well as genes of the λ Red homologous recombination system (y, β, exo genes) under the control of the arabinose-inducible P_{araB} promoter. The plasmid pKD46 is necessary for integration of the PCR product into the chromosome of strain MG1655. The strain MG1655 can be obtained from American Type Culture Collection. (P.O. Box 1549 Manassas, VA 20108, U.S.A.).

Electrocompetent cells were prepared as follows: *E. coli* MG1655/pKD46 was grown overnight at 30 °C in LB medium containing ampicillin (100 mg/l), and the culture was diluted 100 times with 5 ml of SOB medium (Sambrook et al, "Molecular Cloning: A Laboratory Manual, Second Edition", Cold Spring Harbor Laboratory Press, 1989) containing ampicillin and L-arabinose (1 mM). The cells were grown with aeration at 30 °C to an OD₆₀₀ of ≈0.6 and then were made electrocompetent by concentrating 100-fold and washing three times with ice-cold deionized H₂O. Electroporation was performed using 70 ul of cells and ≈100 ng of the PCR product. Cells after electroporation were incubated with 1 ml of SOC medium (Sambrook et al, "Molecular Cloning: A Laboratory Manual, Second Edition", Cold Spring Harbor Laboratory Press, 1989) at 37 °C for 2.5 hours and then were plated onto L-agar containing chloramphenicol (30 μg/ml) and grown at 37 °C to select Cm^{R} recombinants. Then, to eliminate the pKD46 plasmid, two passages on L-agar with Cm at 42 °C were performed and the obtained colonies were tested for sensitivity to ampicillin.

### 2. Verification of deletion of the argT-hisJQMP cluster by PCR

The recombinants in which the *argT*-*hisJQMP* cluster had been deleted and marked with the Cm resistance gene were verified by PCR. Locus-specific primers P3 (SEQ ID NO: 13) and P4 (SEQ ID NO: 14) were used in PCR for the verification. Conditions for PCR verification were as follows: denaturation step for 3 min at 94 °C; profile for 30 cycles: 30 sec at 94 °C, 30 sec at 54 °C, 1 min at 72 °C; final step: 7 min at 72 °C. The PCR product obtained in the reaction with the cells of the recombinant strain as the template was 1650 bp in length. The recombinant strain was named MG1655 Δ argT-hisJQMP::cat.

### Example 2. Production of L-arginine by E. coli 382ΔargT-hisP

To test the effect of inactivation of the *argT-hisJQMP* cluster on L-arginine production, DNA fragments from the chromosome of the above-described *E. coli* MG1655 ΔargT-hisP::cat were transferred to the arginine-producing *E. coli* strain 382 by P1 transduction (Miller, J.H. Experiments in Molecular Genetics, Cold Spring Harbor Lab. Press, 1972, Plainview, NY) to obtain the strain 382ΔargT-hisP. The strain 382 was deposited in the Russian National Collection of Industrial Microorganisms (VKPM) (Russia, 117545 Moscow, 1 Dorozhny proezd, 1) on April 10, 2000 under accession number VKPM B-7926 and then converted to a deposit under the Budapest Treaty on May 18, 2001.

Both *E. coli* strains, 382 and 382ΔargT-hisP, were separately cultivated with shaking at 37 °C for 18 hours in 3 ml of nutrient broth, and 0.3 ml of the obtained cultures were inoculated into 2 ml of a fermentation medium in 20 x 200-mm test tubes and cultivated at 32 °C for 48 hours on a rotary shaker.

After the cultivation, the amount of L-arginine which accumulates in the medium was determined by paper chromatography using the following mobile phase: butanol: acetic acid : water = 4 : 1 : 1 (v/v). A solution of ninhydrin (2%) in acetone was used as a visualizing reagent. A spot containing L-arginine was cut out, L-arginine was eluted with a 0.5% water solution of CdCl₂, and the amount of L-arginine was estimated spectrophotometrically at 540 nm. The results of eight independent test tube fermentations are shown in Table 1. As follows from Table 1, the strain 382ΔargT-hisP produced a larger amount of L- arginine, as compared with 382.

The composition of the fermentation medium (g/l) was as follows:

| | |
|---|---|
| Glucose | 48.0 |
| (NH₄)₂SO₄ | 35.0 |
| KH₂PO₄ | 2.0 |
| MgSO₄·7H₂O | 1.0 |
| Thiamine HCl | 0.0002 |
| Yeast extract | 1.0 |
| L-isoleucine | 0.1 |
| CaCO₃ | 5.0 |

Glucose and magnesium sulfate were sterilized separately. CaCO₃ was dry-heat sterilized at 180 °C for 2 hours. The pH was adjusted to 7.0.

**Table 1**

| Strain | OD₅₄₀ | Amount of L-arginine, g/l |
|---|---|---|
| 382 | 11.3 ± 1.9 | 10.7 ± 1.0 |
| 382ΔargT-hisP | 13.2 ± 1.0 | 11.6 ± 0.7 |

### Example 3. Production of L-lysine by E. coli AJ11442- ΔargT-HisP

To test the effect of inactivation of the *argT-hisJQMP* cluster on lysine production, DNA fragments from the chromosome of the above-described *E. coli* strain MG1655 ΔargT-hisP::cat can be transferred to the lysine-producing *E. coli* strain AJ11442 by P1 transduction (Miller, J.H. Experiments in Molecular Genetics, Cold Spring Harbor Lab. Press, 1972, Plainview, NY) to obtain the AJ114420argT-hisP strain. The strain AJ11442 was deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on May 1, 1981 and received an accession number of FERM P-5084. Then, it was converted to an international deposit under the provisions of the Budapest Treaty on October 29, 1987, and received an accession number of FERM BP-1543.

Both *E. coli* strains, AJ11442 and AJ11442ΔargT-hisP, can be cultured in L-medium containing streptomycin (20 mg/l) at 37 °C, and 0.3 ml of the obtained culture can be inoculated into 20 ml of the fermentation medium containing the required drugs in a 500-ml flask. The cultivation can be carried out at 37 °C for 16 h by using a reciprocal shaker at the agitation speed of 115 rpm. After the cultivation, the amounts of L-lysine and residual glucose in the medium can be measured by a known method (Biotech-analyzer AS210 manufactured by Sakura Seiki Co.). Then, the yield of L-lysine can be calculated relative to consumed glucose for each of the strains.

The composition of the fermentation medium (g/l) is as follows:

| | |
|---|---|
| Glucose | 40 |
| (NH₄)₂SO₄ | 24 |
| K₂HPO₄ | 1.0 |
| MgSO₄ 7H₂O | 1.0 |
| FeSO₄ 7H₂O | 0.01 |
| MnSO₄ 5H₂O | 0.01 |
| Yeast extract | 2.0 |

The pH is adjusted to 7.0 by KOH and the medium is autoclaved at 115 °C for 10 min. Glucose and MgSO₄ 7H₂O are sterilized separately. CaCO₃ is dry-heat sterilized at 180 °C for 2 hours and added to the medium for a final concentration of 30 g/l.

### Example 4. Production of citrulline by E. coli strain 382 ilvA⁺ ΔargG ΔargT-hisP

To test the effect of inactivation of the *argT-hisJQMP* cluster on citrulline production, the citrulline-producing strain 382 ilvA⁺ ΔargG was constructed in the following way.

The strain 382 ilvA⁺ was obtained from the arginine-producing strain 382 (VKPM B-7926) by introducing a wild type *ilvA* gene from E.coli K12 strain by P1 transduction. Clones 382 ilvA⁺ were selected as good growing colonies on minimal agar plates. After that, the citrulline-producing strain 382 ilvA⁺ ΔargG was obtained by deleting the *argG* gene on the chromosome of the 382 ilvA⁺ strain by the method initially developed by Datsenko, K.A. and Wanner, B.L. (Proc. Natl. Acad. Sci. USA, 2000, 97(12), 6640-6645) called "Red-driven integration".

The DNA fragment containing the Cm^{R} marker encoded by the *cat* gene was obtained by PCR, using primers P5 (SEQ ID NO: 15) and P6 (SEQ ID NO: 16), and plasmid pMW118-attL-Cm-attR as the template (WO 05/010175). Primer P5 contains both a region complementary to the region located at the 5' end of the *argG* gene and a region complementary to the attR region. Primer P6 contains both a region complementary to the region located at the 3' end of the *argG* gene and a region complementary to the attL region. The conditions for PCR were as follows: denaturation step for 3 min at 95 °C; profile for two first cycles: 1 min at 95 °C, 30 sec at 50 °C, 40 sec at 72 °C; profile for the last 25 cycles: 30 sec at 95 °C, 30 sec at 54 °C, 40 sec at 72 °C; final step: 5 min at 72 °C.

A 1.85 kbp PCR product was obtained and purified in agarose gel and was used for electroporation of the *E. coli* strain 382 ilvA⁺, which was previously transformed with the plasmid pKD46 which is necessary for integration of the PCR product into the bacterial chromosome. Electrocompetent cells were prepared as described in Example 1. Electroporation was performed using 70 µl of cells and ≈100 ng of the PCR product. Cells after electroporation were incubated and cured from the pKD46 plasmid as described in Example 1.

The recombinants in which the *argG* gene was deleted and marked with the Cm resistance gene were verified by PCR. Locus-specific primers P7 (SEQ ID NO: 17) and P8 (SEQ ID NO: 18) were used in PCR for the verification. Conditions for PCR verification were as follows: denaturation step for 3 min at 94 °C; profile for 30 cycles: 30 sec at 94 °C, 30 sec at 54 °C, 1 min at 72 °C; final step: 7 min at 72 °C. The PCR product obtained in the reaction with the cells of the recombinant strain as the template was 1350 bp in length. The recombinant strain was named 382 ilvA⁺ ΔargG::cat.

Then, the Cm resistance gene (*cat* gene) was eliminated from the chromosome of the *E. coli* 382 ilvA⁺ ΔargG::cat strain using the *int-xis* system. For that purpose *E*. *coli* strain 382 ilvA⁺ ΔargG::cat was transformed with pMW-intxis-ts plasmid (WO 05/010175). The plasmid pMW-intxis-ts carries the gene encoding integrase (Int) of λ phage, and the gene encoding excisionase (Xis), and has temperature-sensitive replicability. Transformant clones were selected on the LB-medium containing 100 µg/ml of ampicillin. Plates were incubated overnight at 30 °C. Transformant clones were cured from the *cat* gene and pMW-intxis-ts plasmid by spreading the separate colonies at 37 °C (at this temperature repressor CIts is partially inactivated and transcription of the *int*/*xis* genes is derepressed) followed by selection of Cm^{S}Ap^{R} variants. Elimination of the *cat* gene from the chromosome of the strain was verified by PCR. Locus-specific primers P7 (SEQ ID NO: 17) and P8 (SEQ ID NO: 18) were used in PCR for the verification. Conditions for PCR verification were as described above. The PCR product obtained in reaction with cells having the eliminated *cat* gene as a template was ∼0.44 kbp in length. Thus, the citrulline-producing strain 382 ilvA⁺ ΔargG was obtained.

To test the effect of inactivation of the *argT-hisJQMP* cluster on citrulline production, DNA fragments from the chromosome of the above-described *E. coli* strain MG1655 ΔargT-hisP::cat was transferred to the *E. coli* citrulline-producing strain 382 ilvA⁺ ΔargG by P1 transduction (Miller, J.H. Experiments in Molecular Genetics, Cold Spring Harbor Lab. Press, 1972, Plainview, NY) to obtain the 382 ilvA⁺ΔargGΔargT-hisP strain.

Both *E. coli* strains, 382 ilvA⁺ ΔargG and 382 ilvA⁺ ΔargG ΔargT-hisP, were separately cultivated with shaking at 37 °C for 18 hours in 3 ml of nutrient broth, and 0.3 ml of the obtained cultures were inoculated into 2 ml of a fermentation medium in 20 x 200-mm test tubes and cultivated at 32 °C for 48 hours on a rotary shaker.

After the cultivation, the amount of citrulline which accumulates in the medium was determined by paper chromatography using the following mobile phase: butanol: acetic acid : water = 4 : 1 : 1 (v/v). A solution of ninhydrin (2%) in acetone can be used as a visualizing reagent. A spot containing citrulline was cut out, citrulline was eluted with 0.5% water solution of CdCl₂, and the amount of citrulline was estimated spectrophotometrically at 540 nm. The results of eight independent test tube fermentations are shown in Table 2. As follows from Table 2, the strain 382 ilvA⁺ ΔargGΔargT-hisP produced a larger amount of L- citrulline, as compared with 382 ilvA⁺ ΔargG.

The composition of the fermentation medium (g/l) can be as follows:

| | |
|---|---|
| Glucose | 48.0 |
| (NH₄)₂SO₄ | 35.0 |
| KH₂PO₄ | 2.0 |
| MgSO₄·7H₂O | 1.0 |
| Thiamine HCl | 0.0002 |
| Yeast extract | 1.0 |
| L-arginine | 0.1 |
| CaCO₃ | 5.0 |

Glucose and magnesium sulfate are sterilized separately. CaCO₃ is dry-heat sterilized at 180 °C for 2 hours. The pH is adjusted to 7.0.

**Table 2**

| Strain | OD₅₄₀ | Amount of L-citrulline, g/l |
|---|---|---|
| 382 ilvA⁺ ΔargG | 12.1±0.3 | 4±0.3 |
| 382 ilvA⁺ ΔargG ΔargT-hisP | 10.4±0.7 | 4.3±0.2 |

### Example 5. Production of ornithine by E. coli strain 382 ΔargFΔargI ΔargT-hisP

To test the effect of inactivation of the *argT-hisJQMP* cluster on ornithine production, DNA fragments from the chromosome of the above-described *E. coli* strain MG1655 ΔargT-hisP::cat can be transferred to the *E. coli* ornithine producing strain 382ΔargFΔargI by P1 transduction (Miller, J.H. Experiments in Molecular Genetics, Cold Spring Harbor Lab. Press, 1972, Plainview, NY) to obtain 382 ΔargFΔargIΔargT-hisP strain. The strain 382ΔargFΔargI can be obtained by consecutive deletion of the *argF* and *argI* genes on the chromosome of the 382 strain (VKPM B-7926) by the method initially developed by Datsenko, K.A. and Wanner, B.L. (Proc. Natl. Acad. Sci. USA, 2000, 97(12), 6640-6645) called "Red-driven integration". According to this procedure, two pairs of PCR primers homologous to both the region adjacent to the *argF* or *argI* gene and the gene which confers antibiotic resistance in the template plasmid can be constructed. The plasmid pMW118-attL-Cm-attR (WO 05/010175) can be used as the template in the PCR reaction.

Both *E. coli* strains, 382ΔargFΔargI and 382ΔargFΔargIΔargT-hisP, can be separately cultivated with shaking at 37 °C for 18 hours in 3 ml of nutrient broth, and 0.3 ml of the obtained cultures were inoculated into 2 ml of a fermentation medium in 20 x 200-mm test tubes and cultivated at 32 °C for 48 hours on a rotary shaker.

After the cultivation, the amount of ornithine which accumulates in the medium can be determined by paper chromatography using the following mobile phase: butanol : acetic acid : water = 4 : 1 : 1 (v/v). A solution of ninhydrin (2%) in acetone can be used as a visualizing reagent. A spot containing ornithine can be cut out, ornithine can be eluted with 0.5% water solution of CdCl₂, and the amount of ornithine can be estimated spectrophotometrically at 540 nm.

The composition of the fermentation medium (g/l) can be as follows:

| | |
|---|---|
| Glucose | 48.0 |
| (NH₄)₂SO₄ | 35.0 |
| KH₂PO₄ | 2.0 |
| MgSO₄·7H₂O | 1.0 |
| Thiamine HCl | 0.0002 |
| Yeast extract | 1.0 |
| L-isoleucine | 0.1 |
| L-arginine | 0.1 |
| CaCO₃ | 5.0 |

Glucose and magnesium sulfate are sterilized separately. CaCO₃ is dry-heat sterilized at 180 °C for 2 hours. The pH is adjusted to 7.0.

### Industrial Applicability

According to the present invention, production of L-amino acid such as L-lysine, L-arginine, ornithine, and citrulline by a bacterium of *Enterobacteriaceae* family can be improved.

## Claims

1. A method for producing an L-amino acid comprising:
- cultivating an L-amino acid-producing bacterium of the *Enterobacteriaceae* family in a medium, and
- collecting said L-amino acid from the medium,
wherein said bacterium has been modified to attenuate expression of one or more genes encoding a lysine/arginine/ornithine transporter, said one or more genes encoding the lysine/arginine/ornithine transporter being one or more genes of *argT-hisJQMP* cluster, and
wherein said bacterium is able to produce and cause accumulation of the L-amino acid in a culture medium in an amount larger than a wild-type or parental strain.

2. The method according to claim 1, wherein said one or more genes of the *argT-hisJQMP* cluster is/are inactivated.

3. The method according to claim 1 or 2, wherein said bacterium belongs to genus *Escherichia.*

4. The method according to claim 3, wherein said bacterium is *Escherichia coli.*

5. The method according to claim 1 or 2, wherein said bacterium belongs to genus *Pantoea.*

6. The method according to any of claims 1 to 5, wherein said L-amino acid is a diaminomonocarboxylic acid.

7. The method according to claim 6, wherein said diaminomonocarboxylic acid is selected from the group consisting of L-lysine, L-arginine, L-ornithine, and L-citrulline.

## Patentansprüche

1. Verfahren zur Produktion einer L-Aminosäure, welches umfasst:
- das Kultivieren eines L-Aminosäure produzierenden Bakteriums der Familie Enterobacteriaceae in einem Medium und
- das Gewinnen der L-Aminosäure aus dem Medium, wobei das Bakterium so modifiziert worden ist, dass die Expression eines oder mehrerer für einen Lysin-/ Arginin-/Ornithin-Transporter kodierenden Gene attenuiert ist, wobei das eine oder die mehreren für einen Lysin-/Arginin-/Ornithin-Transporter kodierenden Gene ein oder mehrere Gene des argT-hisJQMP-Clusters ist/sind, und wobei das Bakterium in der Lage ist, die L-Aminosäure in dem Kulturmedium in einer größeren Menge als ein Wildtypstamm oder Ausgangsstamm zu produzieren und anzuhäufen.

2. Verfahren nach Anspruch 1, wobei das eine oder die mehreren Gene des argT-hisJQMP-Clusters inaktiviert ist/sind.

3. Verfahren nach Anspruch 1 oder 2, wobei das Bakterium zu der Gattung Escherichia gehört.

4. Verfahren nach Anspruch 3, wobei das Bakterium Escherichia coli ist.

5. Verfahren nach Anspruch 1 oder 2, wobei das Bakterium zu der Gattung Pantoea gehört.

6. Verfahren nach einem der Ansprüche 1 bis 5, wobei die L-Aminosäure eine Diaminomonocarbonsäure ist.

7. Verfahren nach Anspruch 6, wobei die Diaminomonocarbonsäure aus der Gruppe ausgewählt ist, die aus L-Lysin, L-Arginin, L-Ornithin und L-Citrullin besteht.

## Revendications

1. Procédé de production d'un acide L-aminé comprenant :
- la culture d'une bactérie produisant un acide L-aminé de la famille des *entérobactériacées* dans un milieu, et
- la collecte dudit acide L-aminé à partir du milieu,
dans lequel ladite bactérie a été modifiée pour atténuer l'expression d'un ou plusieurs gènes codant pour un transporteur de lysine/arginine/ornithine, ledit (lesdits) un ou plusieurs gènes codant pour le transporteur de lysine/arginine/ornithine étant un ou plusieurs gènes du groupe *argT-hisJQMP,* et
dans lequel ladite bactérie est capable de produire l'acide aminé et d'entraîner son accumulation dans un milieu de culture en une quantité supérieure à une souche de type sauvage ou parentale.

2. Procédé selon la revendication 1, dans lequel ledit (lesdits) un ou plusieurs gènes du groupe *argT-hisJQMP* est/sont inactivé(s).

3. Procédé selon la revendication 1 ou 2, dans lequel ladite bactérie appartient au genre *Escherichia.*

4. Procédé selon la revendication 3, dans lequel ladite bactérie est *Escherichia coli.*

5. Procédé selon la revendication 1 ou 2, dans lequel ladite bactérie appartient au genre *Pantoea.*

6. Procédé selon l'une quelconque des revendications 1 à 5, dans lequel ledit acide L-aminé est un acide diaminomonocarboxylique.

7. Procédé selon la revendication 6, dans lequel ledit acide diaminomonocarboxylique est choisi dans le groupe constitué de la L-lysine, de la L-arginine, de la L-ornithine et de la L-citrulline.


### SEQUENCE LISTING

<110> Ajinomoto Co., Inc.
<120> A METHOD FOR PRODUCING AN L-AMINO ACID USING A BACTERIUM OF THE ENTEROBACTERIACEAE FAMILY, HAVING ATTENUATED EXPRESSION OF GENES ENCODING A LYSINE/ARGININE/ORNITHINE TRANSPORTER
<130> D186-11101
<150> RU2010122646
   <151> 2010-06-03
<160> 18
<170> PatentIn version 3.5
<210> 1
   <211> 783
   <212> DNA
   <213> Escherichia coli
<220>
   <221> CDS
   <222> (1)..(783)
<400> 1
   atg aag aag tcg att ctc gct ctg tct ttg tta gtc ggt ctc tcc aca      48
   Met Lys Lys Ser Ile Leu Ala Leu Ser Leu Leu Val Gly Leu Ser Thr
   1               5                   10                  15
   gcg gct tcc agc tat gcg gcg cta ccg gag acg gta cgt atc gga acc      96
   Ala Ala Ser Ser Tyr Ala Ala Leu Pro Glu Thr Val Arg Ile Gly Thr
               20                  25                  30
   gat acc acc tac gca ccg ttc tca tcg aaa gat gct aaa ggt gat ttt     144
   Asp Thr Thr Tyr Ala Pro Phe Ser Ser Lys Asp Ala Lys Gly Asp Phe
           35                  40                  45
   gtt ggc ttt gat atc gat ctc ggt aac gag atg tgc aaa cgg atg cag     192
   Val Gly Phe Asp Ile Asp Leu Gly Asn Glu Met Cys Lys Arg Met Gln
       50                  55                  60
   gtg aaa tgt acc tgg gtt gcc agt gac ttt gac gcg ctg atc ccc tca     240
   Val Lys Cys Thr Trp Val Ala Ser Asp Phe Asp Ala Leu Ile Pro Ser
   65                  70                  75                  80
   ctg aaa gcg aaa aaa atc gac gct att att tcg tcg ctt tcc att acc     288
   Leu Lys Ala Lys Lys Ile Asp Ala Ile Ile Ser Ser Leu Ser Ile Thr
                   85                  90                  95
   gat aaa cgt cag cag gag att gcc ttc tcc gac aag ctg tac gcc gca     336
   Asp Lys Arg Gln Gln Glu Ile Ala Phe Ser Asp Lys Leu Tyr Ala Ala
               100                 105                 110
   gat tct cgt ttg att gcg gcc aaa ggt tca ccg att cag cca acg ctg     384
   Asp Ser Arg Leu Ile Ala Ala Lys Gly Ser Pro Ile Gln Pro Thr Leu
           115                 120                 125
   gat tca ctg aaa ggt aaa cat gtt ggt gtg ctg cag gga tca acc cag     432
   Asp Ser Leu Lys Gly Lys His Val Gly Val Leu Gln Gly Ser Thr Gln
       130                 135                 140
   gaa gct tac gct aac gag acc tgg cgt agt aaa ggc gtg gat gtg gtg     480
   Glu Ala Tyr Ala Asn Glu Thr Trp Arg Ser Lys Gly Val Asp Val Val
   145                 150                 155                 160
   gcc tat gcc aac cag gat ttg gtc tat tcc gat ctg gct gca gga cgt     528
   Ala Tyr Ala Asn Gln Asp Leu Val Tyr Ser Asp Leu Ala Ala Gly Arg
                   165                 170                 175
   ctg gat gct gcg tta caa gat gaa gtt gct gcc agc gaa gga ttc ctc     576
   Leu Asp Ala Ala Leu Gln Asp Glu Val Ala Ala Ser Glu Gly Phe Leu
               180                 185                 190
   aag caa cct gct ggt aaa gat ttc gcc ttt gct ggc tca tca gta aaa     624
   Lys Gln Pro Ala Gly Lys Asp Phe Ala Phe Ala Gly Ser Ser Val Lys
           195                 200                 205
   gac aaa aaa tac ttc ggt gat ggc acc ggt gta ggg cta cgt aaa gat     672
   Asp Lys Lys Tyr Phe Gly Asp Gly Thr Gly Val Gly Leu Arg Lys Asp
       210                 215                 220
   gat gct gaa ctg acg gct gcc ttc aat aag gcg ctt ggc gag ctg cgt     720
   Asp Ala Glu Leu Thr Ala Ala Phe Asn Lys Ala Leu Gly Glu Leu Arg
   225                 230                 235                 240
   cag gac ggc acc tac gac aag atg gcg aaa aag tat ttc gac ttt aat     768
   Gln Asp Gly Thr Tyr Asp Lys Met Ala Lys Lys Tyr Phe Asp Phe Asn
                   245                 250                 255
   gtc tac ggt gac tga                                                 783
   Val Tyr Gly Asp
               260
<210> 2
   <211> 260
   <212> PRT
   <213> Escherichia coli
<400> 2
   Met Lys Lys Ser Ile Leu Ala Leu Ser Leu Leu Val Gly Leu Ser Thr
   1               5                   10                  15
   Ala Ala Ser Ser Tyr Ala Ala Leu Pro Glu Thr Val Arg Ile Gly Thr
               20                  25                  30
   Asp Thr Thr Tyr Ala Pro Phe Ser Ser Lys Asp Ala Lys Gly Asp Phe
           35                  40                  45
   Val Gly Phe Asp Ile Asp Leu Gly Asn Glu Met Cys Lys Arg Met Gln
       50                  55                  60
   Val Lys Cys Thr Trp Val Ala Ser Asp Phe Asp Ala Leu Ile Pro Ser
   65                  70                  75                  80
   Leu Lys Ala Lys Lys Ile Asp Ala Ile Ile Ser Ser Leu Ser Ile Thr
                   85                  90                  95
   Asp Lys Arg Gln Gln Glu Ile Ala Phe Ser Asp Lys Leu Tyr Ala Ala
               100                 105                 110
   Asp Ser Arg Leu Ile Ala Ala Lys Gly Ser Pro Ile Gln Pro Thr Leu
           115                 120                 125
   Asp Ser Leu Lys Gly Lys His Val Gly Val Leu Gln Gly Ser Thr Gln
       130                 135                 140
   Glu Ala Tyr Ala Asn Glu Thr Trp Arg Ser Lys Gly Val Asp Val Val
   145                 150                 155                 160
   Ala Tyr Ala Asn Gln Asp Leu Val Tyr Ser Asp Leu Ala Ala Gly Arg
                   165                 170                 175
   Leu Asp Ala Ala Leu Gln Asp Glu Val Ala Ala Ser Glu Gly Phe Leu
               180                 185                 190
   Lys Gln Pro Ala Gly Lys Asp Phe Ala Phe Ala Gly Ser Ser Val Lys
           195                 200                 205
   Asp Lys Lys Tyr Phe Gly Asp Gly Thr Gly Val Gly Leu Arg Lys Asp
       210                 215                 220
   Asp Ala Glu Leu Thr Ala Ala Phe Asn Lys Ala Leu Gly Glu Leu Arg
   225                 230                 235                 240
   Gln Asp Gly Thr Tyr Asp Lys Met Ala Lys Lys Tyr Phe Asp Phe Asn
                   245                 250                 255
   Val Tyr Gly Asp
               260
<210> 3
   <211> 783
   <212> DNA
   <213> Escherichia coli
<220>
   <221> CDS
   <222> (1)..(783)
<400> 3
   atg aaa aaa ctg gtg cta tcg ctc tct ctg gtt ctg gcc ttc tcc agc        48
   Met Lys Lys Leu Val Leu Ser Leu Ser Leu Val Leu Ala Phe Ser Ser
   1               5                   10                  15
   gca act gcg gcg ttt gct gcg att ccg caa aac atc cgc atc ggt acc        96
   Ala Thr Ala Ala Phe Ala Ala Ile Pro Gln Asn Ile Arg Ile Gly Thr
               20                  25                  30
   gac ccg acc tat gcg cca ttt gaa tca aaa aat tca caa ggc gaa ctg       144
   Asp Pro Thr Tyr Ala Pro Phe Glu Ser Lys Asn Ser Gln Gly Glu Leu
           35                  40                  45
   gtt ggc ttc gat atc gat ctg gca aag gaa tta tgc aaa cgc atc aat       192
   Val Gly Phe Asp Ile Asp Leu Ala Lys Glu Leu Cys Lys Arg Ile Asn
       50                  55                  60
   acg caa tgt acg ttt gtc gaa aat ccg ctg gat gcg tta atc ccg tcc       240
   Thr Gln Cys Thr Phe Val Glu Asn Pro Leu Asp Ala Leu Ile Pro Ser
   65                  70                  75                  80
   tta aaa gcg aag aag att gac gcc atc atg tca tcg ctt tcc att acg       288
   Leu Lys Ala Lys Lys Ile Asp Ala Ile Met Ser Ser Leu Ser Ile Thr
                   85                  90                  95
   gaa aaa cgt cag caa gaa ata gcc ttc acc gac aaa ctg tac gct gcc       336
   Glu Lys Arg Gln Gln Glu Ile Ala Phe Thr Asp Lys Leu Tyr Ala Ala
               100                 105                 110
   gat tct cgt ttg gtg gtg gcg aaa aat tct gac att cag ccg aca gtc       384
   Asp Ser Arg Leu Val Val Ala Lys Asn Ser Asp Ile Gln Pro Thr Val
           115                 120                 125
   gag tcg ctg aaa ggc aaa cgg gta ggc gta ttg cag ggc acc acc cag       432
   Glu Ser Leu Lys Gly Lys Arg Val Gly Val Leu Gln Gly Thr Thr Gln
       130                 135                 140
   gag acg ttc ggt aat gaa cat tgg gca cca aaa ggc att gaa atc gtc       480
   Glu Thr Phe Gly Asn Glu His Trp Ala Pro Lys Gly Ile Glu Ile Val
   145                 150                 155                 160
   tcg tat cag ggg cag gac aac att tat tct gac ctg act gcc gga cgt       528
   Ser Tyr Gln Gly Gln Asp Asn Ile Tyr Ser Asp Leu Thr Ala Gly Arg
                   165                 170                 175
   att gat gcc gcg ttc cag gat gag gtc gct gcc agc gaa ggt ttc ctc       576
   Ile Asp Ala Ala Phe Gln Asp Glu Val Ala Ala Ser Glu Gly Phe Leu
               180                 185                 190
   aaa caa cct gtc ggt aaa gat tac aaa ttc ggt ggc ccg tct gtt aaa       624
   Lys Gln Pro Val Gly Lys Asp Tyr Lys Phe Gly Gly Pro Ser Val Lys
           195                 200                 205
   gat gaa aaa ctg ttt ggc gta ggg acc ggc atg ggc ctg cgt aaa gaa       672
   Asp Glu Lys Leu Phe Gly Val Gly Thr Gly Met Gly Leu Arg Lys Glu
       210                 215                 220
   gat aac gaa ctg cgc gaa gca ctg aac aaa gcc ttt gcc gaa atg cgc       720
   Asp Asn Glu Leu Arg Glu Ala Leu Asn Lys Ala Phe Ala Glu Met Arg
   225                 230                 235                 240
   gct gac ggt act tac gag aaa tta gcg aaa aag tac ttc gat ttt gat       768
   Ala Asp Gly Thr Tyr Glu Lys Leu Ala Lys Lys Tyr Phe Asp Phe Asp
                   245                 250                 255
   gtt tat ggt ggc taa                                                   783
   Val Tyr Gly Gly
               260
<210> 4
   <211> 260
   <212> PRT
   <213> Escherichia coli
<400> 4
   Met Lys Lys Leu Val Leu Ser Leu Ser Leu Val Leu Ala Phe Ser Ser
   1               5                   10                  15
   Ala Thr Ala Ala Phe Ala Ala Ile Pro Gln Asn Ile Arg Ile Gly Thr
               20                  25                  30
   Asp Pro Thr Tyr Ala Pro Phe Glu Ser Lys Asn Ser Gln Gly Glu Leu
           35                  40                  45
   Val Gly Phe Asp Ile Asp Leu Ala Lys Glu Leu Cys Lys Arg Ile Asn
       50                  55                  60
   Thr Gln Cys Thr Phe Val Glu Asn Pro Leu Asp Ala Leu Ile Pro Ser
   65                  70                  75                  80
   Leu Lys Ala Lys Lys Ile Asp Ala Ile Met Ser Ser Leu Ser Ile Thr
                   85                  90                  95
   Glu Lys Arg Gln Gln Glu Ile Ala Phe Thr Asp Lys Leu Tyr Ala Ala
               100                 105                 110
   Asp Ser Arg Leu Val Val Ala Lys Asn Ser Asp Ile Gln Pro Thr Val
           115                 120                 125
   Glu Ser Leu Lys Gly Lys Arg Val Gly Val Leu Gln Gly Thr Thr Gln
       130                 135                 140
   Glu Thr Phe Gly Asn Glu His Trp Ala Pro Lys Gly Ile Glu Ile Val
   145                 150                 155                 160
   Ser Tyr Gln Gly Gln Asp Asn Ile Tyr Ser Asp Leu Thr Ala Gly Arg
                   165                 170                 175
   Ile Asp Ala Ala Phe Gln Asp Glu Val Ala Ala Ser Glu Gly Phe Leu
               180                 185                 190
   Lys Gln Pro Val Gly Lys Asp Tyr Lys Phe Gly Gly Pro Ser Val Lys
           195                 200                 205
   Asp Glu Lys Leu Phe Gly Val Gly Thr Gly Met Gly Leu Arg Lys Glu
       210                 215                 220
   Asp Asn Glu Leu Arg Glu Ala Leu Asn Lys Ala Phe Ala Glu Met Arg
   225                 230                 235                 240
   Ala Asp Gly Thr Tyr Glu Lys Leu Ala Lys Lys Tyr Phe Asp Phe Asp
                   245                 250                 255
   Val Tyr Gly Gly
               260
<210> 5
   <211> 687
   <212> DNA
   <213> Escherichia coli
<220>
   <221> CDS
   <222> (1)..(687)
<400> 5
   atg ttg tat ggg ttt tca ggt gtt att tta cag ggt gcg ctc gtc acg        48
   Met Leu Tyr Gly Phe Ser Gly Val Ile Leu Gln Gly Ala Leu Val Thr
   1               5                   10                  15
   ctg gag ctg gct atc agc tct gta gtg ctc gct gta atc atc ggt tta        96
   Leu Glu Leu Ala Ile Ser Ser Val Val Leu Ala Val Ile Ile Gly Leu
               20                  25                  30
   att ggc gct ggc ggt aag ctc tcg caa aat cgg ctt tcg ggg ctg att       144
   Ile Gly Ala Gly Gly Lys Leu Ser Gln Asn Arg Leu Ser Gly Leu Ile
           35                  40                  45
   ttc gaa ggg tac acc acg ctg att cgt ggc gtg ccg gac tta gtg ttg       192
   Phe Glu Gly Tyr Thr Thr Leu Ile Arg Gly Val Pro Asp Leu Val Leu
       50                  55                  60
   atg ctg ctg att ttc tac ggt ttg cag att gcg cta aac acg gtg acg       240
   Met Leu Leu Ile Phe Tyr Gly Leu Gln Ile Ala Leu Asn Thr Val Thr
   65                  70                  75                  80
   gag gcg atg ggc gtc ggg cag att gat atc gat ccg atg gtc gct ggt       288
   Glu Ala Met Gly Val Gly Gln Ile Asp Ile Asp Pro Met Val Ala Gly
                   85                  90                  95
   att atc act ctc ggt ttt atc tac ggt gct tat ttt acc gaa acg ttc       336
   Ile Ile Thr Leu Gly Phe Ile Tyr Gly Ala Tyr Phe Thr Glu Thr Phe
               100                 105                 110
   cgt ggt gct ttt atg gca gtg ccg aaa gga cat ata gag gcg gcg acg       384
   Arg Gly Ala Phe Met Ala Val Pro Lys Gly His Ile Glu Ala Ala Thr
           115                 120                 125
   gcg ttc ggt ttt act cgt ggg caa gtg ttt cgg cgg atc atg ttt ccg       432
   Ala Phe Gly Phe Thr Arg Gly Gln Val Phe Arg Arg Ile Met Phe Pro
       130                 135                 140
   tcg atg atg cgt tac gcc ctg cca ggc att ggc aac aac tgg cag gtg       480
   Ser Met Met Arg Tyr Ala Leu Pro Gly Ile Gly Asn Asn Trp Gln Val
   145                 150                 155                 160
   atc ctc aaa tct acc gca ctg gtt tcg tta ctc ggc ctg gaa gat gtg       528
   Ile Leu Lys Ser Thr Ala Leu Val Ser Leu Leu Gly Leu Glu Asp Val
                   165                 170                 175
   gtc aaa gcc acc caa ctg gca ggc aaa agt acc tgg gaa ccg ttc tat       576
   Val Lys Ala Thr Gln Leu Ala Gly Lys Ser Thr Trp Glu Pro Phe Tyr
               180                 185                 190
   ttc gcc atc gtc tgt ggc gtg att tac ctg gtt ttc acc act gtt tcc       624
   Phe Ala Ile Val Cys Gly Val Ile Tyr Leu Val Phe Thr Thr Val Ser
           195                 200                 205
   aat ggt gtg ctg ctg ttc ctt gag cgc cgc tac tcc gtg ggt gtg aag       672
   Asn Gly Val Leu Leu Phe Leu Glu Arg Arg Tyr Ser Val Gly Val Lys
       210                 215                 220
   agg gct gac ctg tga                                                   687
   Arg Ala Asp Leu
   225
<210> 6
   <211> 228
   <212> PRT
   <213> Escherichia coli
<400> 6
   Met Leu Tyr Gly Phe Ser Gly Val Ile Leu Gln Gly Ala Leu Val Thr
   1               5                   10                  15
   Leu Glu Leu Ala Ile Ser Ser Val Val Leu Ala Val Ile Ile Gly Leu
               20                  25                  30
   Ile Gly Ala Gly Gly Lys Leu Ser Gln Asn Arg Leu Ser Gly Leu Ile
           35                  40                  45
   Phe Glu Gly Tyr Thr Thr Leu Ile Arg Gly Val Pro Asp Leu Val Leu
       50                  55                  60
   Met Leu Leu Ile Phe Tyr Gly Leu Gln Ile Ala Leu Asn Thr Val Thr
   65                  70                  75                  80
   Glu Ala Met Gly Val Gly Gln Ile Asp Ile Asp Pro Met Val Ala Gly
                   85                  90                  95
   Ile Ile Thr Leu Gly Phe Ile Tyr Gly Ala Tyr Phe Thr Glu Thr Phe
               100                 105                 110
   Arg Gly Ala Phe Met Ala Val Pro Lys Gly His Ile Glu Ala Ala Thr
           115                 120                 125
   Ala Phe Gly Phe Thr Arg Gly Gln Val Phe Arg Arg Ile Met Phe Pro
       130                 135                 140
   Ser Met Met Arg Tyr Ala Leu Pro Gly Ile Gly Asn Asn Trp Gln Val
   145                 150                 155                 160
   Ile Leu Lys Ser Thr Ala Leu Val Ser Leu Leu Gly Leu Glu Asp Val
                   165                 170                 175
   Val Lys Ala Thr Gln Leu Ala Gly Lys Ser Thr Trp Glu Pro Phe Tyr
               180                 185                 190
   Phe Ala Ile Val Cys Gly Val Ile Tyr Leu Val Phe Thr Thr Val Ser
           195                 200                 205
   Asn Gly Val Leu Leu Phe Leu Glu Arg Arg Tyr Ser Val Gly Val Lys
       210                 215                 220
   Arg Ala Asp Leu
   225
<210> 7
   <211> 717
   <212> DNA
   <213> Escherichia coli
<220>
   <221> CDS
   <222> (1)..(717)
<400> 7
   gtg atc gaa atc tta cat gaa tac tgg aaa ccg ctg ctg tgg acc gac        48
   Val Ile Glu Ile Leu His Glu Tyr Trp Lys Pro Leu Leu Trp Thr Asp
   1               5                   10                  15
   ggt tat cgc ttt act ggt gtg gcg atc act ctg tgg ctg ctt att ttg        96
   Gly Tyr Arg Phe Thr Gly Val Ala Ile Thr Leu Trp Leu Leu Ile Leu
               20                  25                  30
   tcg gta gtg ata ggc gga gtc ctg gcg ctg ttt ctg gcg att ggt cgt       144
   Ser Val Val Ile Gly Gly Val Leu Ala Leu Phe Leu Ala Ile Gly Arg
           35                  40                  45
   gtc tcc agt aat aaa tac atc cag ttt cca atc tgg tta ttt acc tat       192
   Val Ser Ser Asn Lys Tyr Ile Gln Phe Pro Ile Trp Leu Phe Thr Tyr
       50                  55                  60
   att ttt cgc ggt acg ccg ctg tat gtt cag ttg ctg gtg ttc tat tcc       240
   Ile Phe Arg Gly Thr Pro Leu Tyr Val Gln Leu Leu Val Phe Tyr Ser
   65                  70                  75                  80
   ggc atg tac acg ctt gag att gtt aag gga acc gaa ttc ctt aac gct       288
   Gly Met Tyr Thr Leu Glu Ile Val Lys Gly Thr Glu Phe Leu Asn Ala
                   85                  90                  95
   ttc ttc cgc agt ggc ctg aac tgt acc gtg ctg gcg ctg acg ctt aac       336
   Phe Phe Arg Ser Gly Leu Asn Cys Thr Val Leu Ala Leu Thr Leu Asn
               100                 105                 110
   acc tgc gct tac act acc gag att ttt gct ggg gca atc cgt tcg gtt       384
   Thr Cys Ala Tyr Thr Thr Glu Ile Phe Ala Gly Ala Ile Arg Ser Val
           115                 120                 125
   ccg cat ggg gaa att gaa gcc gcc aga gcc tat ggc ttc tcg act ttt       432
   Pro His Gly Glu Ile Glu Ala Ala Arg Ala Tyr Gly Phe Ser Thr Phe
       130                 135                 140
   aaa atg tat cgc tgc att att ttg cct tct gcg ctg cgt att gcg tta       480
   Lys Met Tyr Arg Cys Ile Ile Leu Pro Ser Ala Leu Arg Ile Ala Leu
   145                 150                 155                 160
   ccg gca tac agc aac gaa gtg atc ctg atg ctg cac tct act gcg ttg       528
   Pro Ala Tyr Ser Asn Glu Val Ile Leu Met Leu His Ser Thr Ala Leu
                   165                 170                 175
   gca ttt act gcc acg gtg ccg gat ctg ctg aaa ata gcc cgc gat att       576
   Ala Phe Thr Ala Thr Val Pro Asp Leu Leu Lys Ile Ala Arg Asp Ile
               180                 185                 190
   aac gcc gcc acg tat caa cct ttt acc gcc ttc ggc att gcc gcg gtg       624
   Asn Ala Ala Thr Tyr Gln Pro Phe Thr Ala Phe Gly Ile Ala Ala Val
           195                 200                 205
   ctc tat tta atc atc tct tat gtc ctg atc agc ctc ttt cgc aga gcg       672
   Leu Tyr Leu Ile Ile Ser Tyr Val Leu Ile Ser Leu Phe Arg Arg Ala
       210                 215                 220
   gaa aaa cgc tgg ttg cag cat gtg aaa cct tct tca acg cac tga           717
   Glu Lys Arg Trp Leu Gln His Val Lys Pro Ser Ser Thr His
   225                 230                 235
<210> 8
   <211> 238
   <212> PRT
   <213> Escherichia coli
<400> 8
   Val Ile Glu Ile Leu His Glu Tyr Trp Lys Pro Leu Leu Trp Thr Asp
   1               5                   10                  15
   Gly Tyr Arg Phe Thr Gly Val Ala Ile Thr Leu Trp Leu Leu Ile Leu
               20                  25                  30
   Ser Val Val Ile Gly Gly Val Leu Ala Leu Phe Leu Ala Ile Gly Arg
           35                  40                  45
   Val Ser Ser Asn Lys Tyr Ile Gln Phe Pro Ile Trp Leu Phe Thr Tyr
       50                  55                  60
   Ile Phe Arg Gly Thr Pro Leu Tyr Val Gln Leu Leu Val Phe Tyr Ser
   65                  70                  75                  80
   Gly Met Tyr Thr Leu Glu Ile Val Lys Gly Thr Glu Phe Leu Asn Ala
                   85                  90                  95
   Phe Phe Arg Ser Gly Leu Asn Cys Thr Val Leu Ala Leu Thr Leu Asn
               100                 105                 110
   Thr Cys Ala Tyr Thr Thr Glu Ile Phe Ala Gly Ala Ile Arg Ser Val
           115                 120                 125
   Pro His Gly Glu Ile Glu Ala Ala Arg Ala Tyr Gly Phe Ser Thr Phe
       130                 135                 140
   Lys Met Tyr Arg Cys Ile Ile Leu Pro Ser Ala Leu Arg Ile Ala Leu
   145                 150                 155                 160
   Pro Ala Tyr Ser Asn Glu Val Ile Leu Met Leu His Ser Thr Ala Leu
                   165                 170                 175
   Ala Phe Thr Ala Thr Val Pro Asp Leu Leu Lys Ile Ala Arg Asp Ile
               180                 185                 190
   Asn Ala Ala Thr Tyr Gln Pro Phe Thr Ala Phe Gly Ile Ala Ala Val
           195                 200                 205
   Leu Tyr Leu Ile Ile Ser Tyr Val Leu Ile Ser Leu Phe Arg Arg Ala
       210                 215                 220
   Glu Lys Arg Trp Leu Gln His Val Lys Pro Ser Ser Thr His
   225                 230                 235
<210> 9
   <211> 774
   <212> DNA
   <213> Escherichia coli
<220>
   <221> CDS
   <222> (1)..(774)
<400> 9
   atg tcc gag aat aaa tta aac gtt atc gat ttg cac aaa cgc tac ggc      48
   Met Ser Glu Asn Lys Leu Asn Val Ile Asp Leu His Lys Arg Tyr Gly
   1               5                   10                  15
   gaa cat gaa gtg ctg aaa ggg gta tca ctg caa gcg aat gcc gga gat      96
   Glu His Glu Val Leu Lys Gly Val Ser Leu Gln Ala Asn Ala Gly Asp
               20                  25                  30
   gta ata agc atc atc gga tcg tcg gga tcg ggg aaa agt acc ttt ctg      144
   Val Ile Ser Ile Ile Gly Ser Ser Gly Ser Gly Lys Ser Thr Phe Leu
           35                  40                  45
   cgc tgc att aac ttc ctc gaa aaa ccg agt gaa ggg tcg atc gtg gtc      192
   Arg Cys Ile Asn Phe Leu Glu Lys Pro Ser Glu Gly Ser Ile Val Val
       50                  55                  60
   aat ggc cag acg atc aat ctg gtg cgc gac aaa gac ggt caa ctc aaa      240
   Asn Gly Gln Thr Ile Asn Leu Val Arg Asp Lys Asp Gly Gln Leu Lys
   65                  70                  75                  80
   gtc gcc gat aaa aat caa ctg cgc tta ctg cgc aca cgc ctg acg atg      288
   Val Ala Asp Lys Asn Gln Leu Arg Leu Leu Arg Thr Arg Leu Thr Met
                   85                  90                  95
   gta ttc cag cac ttc aat ctc tgg agc cat atg acg gtg ctg gaa aac      336
   Val Phe Gln His Phe Asn Leu Trp Ser His Met Thr Val Leu Glu Asn
               100                 105                 110
   gtc atg gaa gcg ccg att cag gtg ttg ggc ctg agc aag cag gaa gcg      384
   Val Met Glu Ala Pro Ile Gln Val Leu Gly Leu Ser Lys Gln Glu Ala
           115                 120                 125
   cgc gag cgg gcg gtg aag tat ctg gca aaa gtc ggg ata gac gaa cgt      432
   Arg Glu Arg Ala Val Lys Tyr Leu Ala Lys Val Gly Ile Asp Glu Arg
       130                 135                 140
   gcg cag ggg aaa tat ccg gtg cat ctt tcc ggc ggt cag caa cag cgt      480
   Ala Gln Gly Lys Tyr Pro Val His Leu Ser Gly Gly Gln Gln Gln Arg
   145                 150                 155                 160
   gtt tct atc gcg cgg gcg ctg gcg atg gaa ccg gaa gtt tta ctg ttt      528
   Val Ser Ile Ala Arg Ala Leu Ala Met Glu Pro Glu Val Leu Leu Phe
                   165                 170                 175
   gat gaa cct acc tcg gcg ctc gat cct gaa ctg gta ggc gaa gtg ttg      576
   Asp Glu Pro Thr Ser Ala Leu Asp Pro Glu Leu Val Gly Glu Val Leu
               180                 185                 190
   cgt att atg cag caa ctg gca gaa gag ggg aaa acc atg gtg gta gtg      624
   Arg Ile Met Gln Gln Leu Ala Glu Glu Gly Lys Thr Met Val Val Val
           195                 200                 205
   act cac gaa atg ggc ttt gct cgc cat gtt tct act cat gtc att ttc      672
   Thr His Glu Met Gly Phe Ala Arg His Val Ser Thr His Val Ile Phe
       210                 215                 220
   ctc cat cag ggg aaa ata gaa gaa gag ggc gcg ccg gag cag tta ttt      720
   Leu His Gln Gly Lys Ile Glu Glu Glu Gly Ala Pro Glu Gln Leu Phe
   225                 230                 235                 240
   ggc aac ccg caa agc cct cgt ctg caa cgg ttc ctt aag gga tcg ctg      768
   Gly Asn Pro Gln Ser Pro Arg Leu Gln Arg Phe Leu Lys Gly Ser Leu
                   245                 250                 255
   aaa taa                                                              774
   Lys
<210> 10
   <211> 257
   <212> PRT
   <213> Escherichia coli
<400> 10
   Met Ser Glu Asn Lys Leu Asn Val Ile Asp Leu His Lys Arg Tyr Gly
   1               5                   10                  15
   Glu His Glu Val Leu Lys Gly Val Ser Leu Gln Ala Asn Ala Gly Asp
               20                  25                  30
   Val Ile Ser Ile Ile Gly Ser Ser Gly Ser Gly Lys Ser Thr Phe Leu
           35                  40                  45
   Arg Cys Ile Asn Phe Leu Glu Lys Pro Ser Glu Gly Ser Ile Val Val
       50                  55                  60
   Asn Gly Gln Thr Ile Asn Leu Val Arg Asp Lys Asp Gly Gln Leu Lys
   65                  70                  75                  80
   Val Ala Asp Lys Asn Gln Leu Arg Leu Leu Arg Thr Arg Leu Thr Met
                   85                  90                  95
   Val Phe Gln His Phe Asn Leu Trp Ser His Met Thr Val Leu Glu Asn
               100                 105                 110
   Val Met Glu Ala Pro Ile Gln Val Leu Gly Leu Ser Lys Gln Glu Ala
           115                 120                 125
   Arg Glu Arg Ala Val Lys Tyr Leu Ala Lys Val Gly Ile Asp Glu Arg
       130                 135                 140
   Ala Gln Gly Lys Tyr Pro Val His Leu Ser Gly Gly Gln Gln Gln Arg
   145                 150                 155                 160
   Val Ser Ile Ala Arg Ala Leu Ala Met Glu Pro Glu Val Leu Leu Phe
                   165                 170                 175
   Asp Glu Pro Thr Ser Ala Leu Asp Pro Glu Leu Val Gly Glu Val Leu
               180                 185                 190
   Arg Ile Met Gln Gln Leu Ala Glu Glu Gly Lys Thr Met Val Val Val
           195                 200                 205
   Thr His Glu Met Gly Phe Ala Arg His Val Ser Thr His Val Ile Phe
       210                 215                 220
   Leu His Gln Gly Lys Ile Glu Glu Glu Gly Ala Pro Glu Gln Leu Phe
   225                 230                 235                 240
   Gly Asn Pro Gln Ser Pro Arg Leu Gln Arg Phe Leu Lys Gly Ser Leu
                   245                 250                 255
   Lys
<210> 11
   <211> 64
   <212> DNA
   <213> Artificial Sequence
<220>
   <223> Primer P1
<400> 11
   gttatgtatg aagaagtcga ttctcgctct gtctttcgct caagttagta taaaaaagct
60
gaac
64
<210> 12
   <211> 64
   <212> DNA
   <213> Artificial Sequence
<220>
   <223> Primer P2
<400> 12
   tactccggca gcgttagcca ccggagagga aattattgaa gcctgccttt ttatactaag    60
   ttgg                                                                 64
<210> 13
   <211> 20
   <212> DNA
   <213> Artificial Sequence
<220>
   <223> Primer P3
<400> 13
   tgtcgtcaag atggcataag 20
<210> 14
   <211> 21
   <212> DNA
   <213> Artificial Sequence
<220>
   <223> Primer P4
<400> 14
   aacacccaat gcagcaggca g 21
<210> 15
   <211> 64
   <212> DNA
   <213> Artificial Sequence
<220>
   <223> Primer P5
<400> 15
   tcagttaatt aagcagggtg ttattttatg acgacgcgct caagttagta taaaaaagct    60
   gaac                                                                 64
<210> 16
   <211> 64
   <212> DNA
   <213> Artificial Sequence
<220>
   <223> Primer P6
<400> 16
   gttgatgtcg aattactggc ctttgttttc cagatttgaa gcctgccttt ttatactaag    60
   ttgg                                                                 64
<210> 17
   <211> 27
   <212> DNA
   <213> Artificial Sequence
<220>
   <223> Primer P7
<400> 17
   tagtcgactg caggcattat agtgatc 27
<210> 18
   <211> 30
   <212> DNA
   <213> Artificial Sequence
<220>
   <223> Primer P8
<400> 18
   attctagaag cttggatgca aaccatgatg 30